# EUROPEAN PATENT APPLICATION

(11) **EP 2 075 329 A1**
(43) Date of publication of application: **01.07.2009**
(21) Application number: 07828282.9
(22) Date of filing: 20.09.2007
(51) Int. Cl.: C12N 5/00, A61L 27/00, C12N 5/06

(54) **COMPOSITION FOR REGENERATION OF PERIODONTAL HARD TISSUE AND METHOD FOR REGENERATION OF PERIODONTAL HARD TISSUE**

(30) Priority: 25.09.2006 JP 2006259717
(71) Applicant: Osaka University, Suita-shi Osaka 565-0871 (JP)
(72) Inventor: MURAKAMI, Shinya, Suita-shi Osaka 565-0871 (JP); HASHIKAWA, Tomoko, Suita-shi Osaka 565-0871 (JP); SAWA, Yoshiki, Suita-shi Osaka 565-0871 (JP); MATSUYAMA, Akifumi, Suita-shi Osaka 565-0871 (JP); KOMODA, Hiroshi, Suita-shi Osaka 565-0871 (JP)
(74) Representative: Smyth, Gyles Darren
(86) International application number: PCT/JP2007/068287
(87) International publication number: WO 2008/038572

(57) **Abstract**

An object of the present invention is to provide technology of using a preadipocyte to restore comprehensively bone, cartilage and periodontal ligament, and cause a periodontal hard tissue to regenerate. By culturing a preadipocyte using a culture medium containing (i) at least one member selected from the group consisting of glycerophosphate and salts thereof, and (ii) at least one member selected from the group consisting of ascorbic acid, derivatives thereof and salts thereof and not containing an adrenal cortical hormone in such an amount that promotes differentiation of a preadipocyte into a periodontal hard tissue cell, differentiation of the cell into a periodontal hard tissue cell is induced efficiently. Furthermore, a scaffold is combined with the preadipocyte cultured with the above-mentioned culture medium to be transplanted into a periodontal tissue that requires regeneration.

## Description

### TECHNICAL FIELD

The present invention relates to a culture method for causing preadipocytes to differentiate into a periodontal hard tissue. Furthermore, the present invention relates to a composition for periodontal hard tissue regeneration that is used in order to cause a periodontal hard tissue to regenerate, including bone, cartilage and periodontal ligament. Furthermore, the present invention relates to a therapeutic method for periodontal diseases.

### BACKGROUND ART

In recent years, in the treatment of periodontal diseases which are accompanied by the destruction of periodontal hard tissue including alveolar bone, cementum and periodontal membrane, advances have been made in approaches that devise regeneration by activating the self-repairing ability of endogenous tissue-derived stem cells by some method (scaffold, cell growth factor or the like). Meanwhile, regenerative treatments, which cell-transplant into periodontal tissue stem cells, beginning with hematopoietic stem cells, mesenchymal line stem cells and ES (pluripotent stem cells), are also gathering attention as therapeutic methods for the periodontal diseases mentioned above.

Although stem cells are also drawing attention as cell source for regenerative medicine due to having self-replication capability as well as differentiation capability to differentiate into many species of cells, details of practicality and usefulness thereof have not yet been clarified sufficiently. In addition, a periodontal hard tissue is constituted by a plurality of tissues, such as, alveolar bone, cementum, cartilage and periodontal ligament, such that when a periodontal hard tissue is damaged, high-level regenerative medicine techniques, which restore these tissues comprehensively, are required.

In addition, as stem cell sources for regenerative medicine those collected from bone marrow are used widely in prior art. However, collection of stem cells from bone marrow is considerably invasive to the body, and, in addition, there is the problem that the number of cells that can be collected is limited. In recent years, fat tissue-derived preadipocytes have been reported as alternative cells to such bone marrow-derived stem cells. The present inventors have confirmed that preadipocytes not only have the advantages of adding little burden to the body when collected and excellent multiplication ability, but also have the ability to differentiate into bone and cartilage.

However, concrete technical means for using preadipocytes to cause a periodontal hard tissue to regenerate efficiently and effectively have not been clarified. In addition, to induce differentiation of mesenchymal stem cells into bone or cartilage, culture in a culture medium containing an adrenal cortical hormone such as dexamethasone has been adopted in prior art; however, it has been shown by the present inventors that preadipocytes cannot differentiate efficiently into periodontal hard tissue with such methods.
Patent Reference 1
   Published Japanese Translation of a PCT Application No. 2002-537849

### DISCLOSURE OF THE INVENTION

An object of the present invention is to provide a technique for using a preadipocyte to restore comprehensively bone, cartilage and periodontal ligament to cause a periodontal hard tissue to regenerate.

The present inventors carried out earnest studies to resolve the above issues and discovered that by culturing a preadipocyte using a culture medium containing (i) at least one member selected from the group consisting of glycerophosphate and salts thereof and (ii) at least one member selected from the group consisting of ascorbic acid, derivatives thereof and salts thereof, and not containing an adrenal cortical hormone in such an amount that promotes differentiation of a preadipocyte into a periodontal hard tissue cell, differentiation of the cell into a periodontal hard tissue cell can be induced efficiently. Furthermore, the inventors have discovered that combining a scaffold together with preadipocytes cultured with the above-mentioned culture medium for transplantation into a periodontal tissue that needs to be regenerated, allowed comprehensively bone, cartilage and periodontal ligament to be restored, regenerating a periodontal hard tissue. The inventors have discovered that such regeneration effect of periodontal hard tissue could be increased all the more in particular by selecting hydroxyapatite and/or fibrin gel as scaffold and useing this in combination with the preadipocytes cultured in the above-mentioned culture medium. The present invention has been completed by further rounds of improvement based on these observations.

That is to say, the present invention provides the following culture methods, compositions for periodontal hard tissue regeneration, therapeutic methods for periodontal disease, and uses.

Item 1. A culture method for causing a preadipocyte to differentiate into a periodontal hard tissue cell, wherein a preadipocyte is cultured with a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such a amount that promotes differentiation of a preadipocyte into a periodontal hard tissue cell.

Item 2. The method according to Item 1, wherein the preadipocyte is human-derived.

Item 3. The method according to Item 1, wherein Component (i) is β glycerophosphate and Component (ii) is ascorbic acid.

Item 4. The method according to Item 1, wherein the culture medium contains 0.1 to 100mg/mL of Component (i) and 1 to 1000µg/mL of Component (ii).

Item 5. The method according to Item 1, wherein the culture medium contains the adrenal cortical hormone in an amount of less than 10nM.

Item 6. A composition for periodontal hard tissue regeneration, containing a scaffold, and preadipocytes cultured with a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such a amount that promotes differentiation of a preadipocyte into a periodontal hard tissue cell.

Item 7. The composition for periodontal hard tissue regeneration according to Item 6, wherein the scaffold is hydroxyapatite and/or fibrin gel.

Item 8. The composition for periodontal hard tissue regeneration according to Item 6, wherein the preadipocyte is human-derived.

Item 9. The composition for periodontal hard tissue regeneration according to Item 6, containing 1 to 4000mg of the scahold per 1.0×10⁶ cells of the preadipocytes.

Item 10. A therapeutic method for periodontal disease, comprising administering a therapeutically effective amount of a caffold and preadipocytes cultured with a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such an amount that promotes differentiation of a preadipocyte into a periodontal hard tissue cell to a periodontal-diseased area of a patient affected by a periodontal disease.

Item 11. The therapeutic method for periodontal disease according to Item 10, wherein a therapeutically effective amount of the composition for periodontal hard tissue regeneration of Item 6 is administered to a periodontal-diseased area of a patient affected by a periodontal disease.

Item 12. Use of a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such a amount that promotes differentiation of a preadipocyte into a periodontal hard tissue cell, for causing a preadipocyte to differentiate into a periodontal hard tissue cell.

Item 13. Use of a scaffold and preadipocyte cultured with a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such a amount that promotes differentiation of a preadipocyte into a periodontal hard tissue cell, for preparing a composition for periodontal hard tissue regeneration.

### EFFECT OF THE INVENTION

According to the culture method of the present invention, a preadipocyte obtained from fat tissue can be caused to induce differentiation efficiently into a cell constituting a periodontal hard tissue.

In addition, according to the composition for periodontal hard tissue regeneration of the present invention, combining and using preadipocytes obtained from a fat tissue and a scaffold allows a bone, a cartilage or a periodontal ligament to be restored comprehensively to regenerate a periodontal hard tissue. In particular, such effect of regenerating a periodontal hard tissue is demonstrated all the more remarkably by adopting hydroxyapatite and/or fibrin gel as a scaffold.

Consequently, according to the composition for periodontal hard tissue regeneration of the present invention, regeneration of periodontal hard tissue can be carried out efficiently also against severe periodontal infections accompanied by the destruction of alveolar bone, cementum or periodontal ligament, from which recovery was difficult in prior art. That is to say, according to the present invention, a novel therapeutic method contributing to the improvement of a patient's quality of life (QOL) can be provided, which is important good news for patients, and thus contributing to the improvement of people's well-being.

### BRIEF DESCRIPTION OF THE DRAWINGS

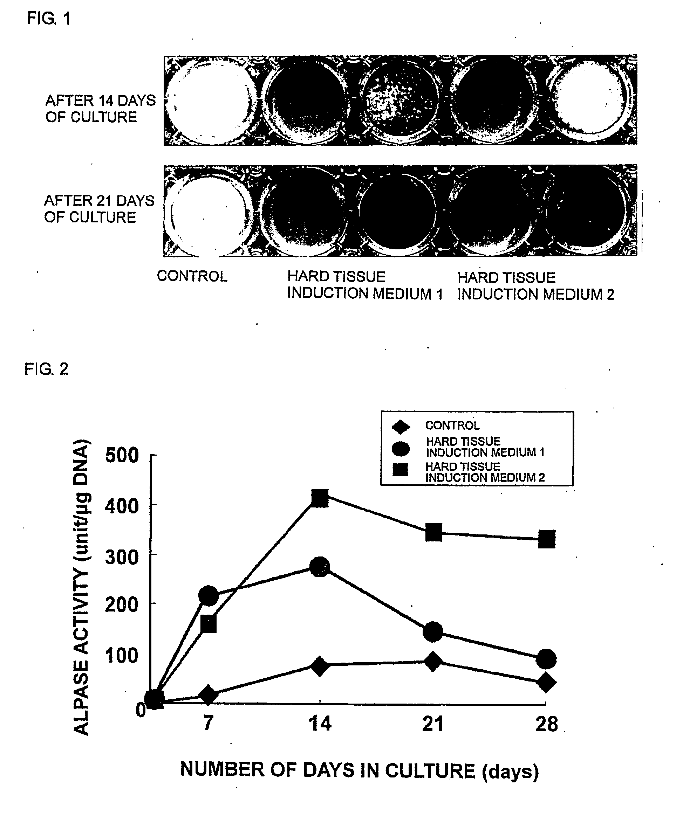

Fig. 1 is a photograph of cells stained with alizarin red after culture in Test Example 1;
Fig. 2 is a figure showing the results of measurements of the amount of ALPase expression for cells after culture in Test Example 1;

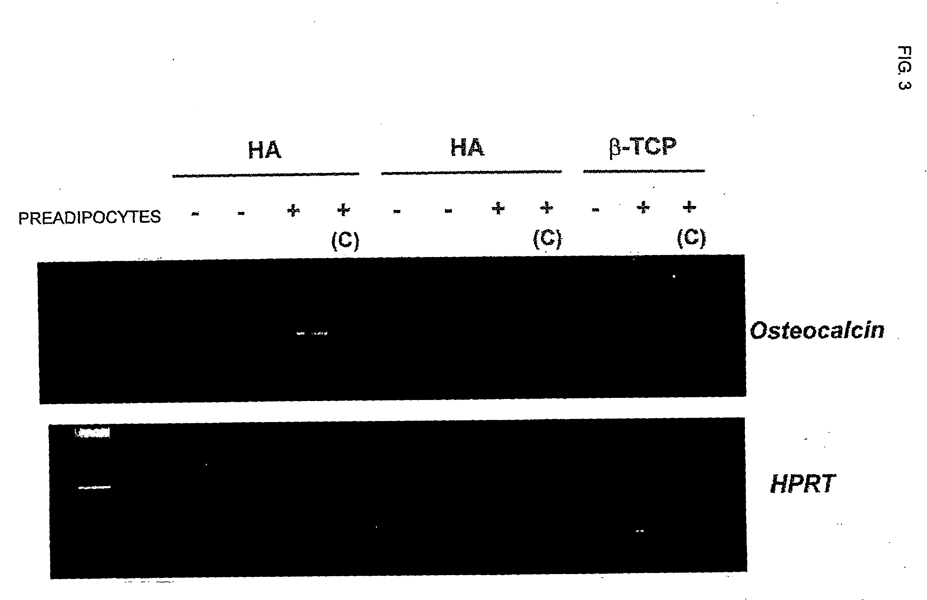

Fig. 3 shows the results of measurements by the RT-PCR method, after transplanting preadipocyte and hydroxyapatite (HA) or β-TCP under the dorsal skin of mouse, of the expression of human osteocalcin (differentiation marker for osteoblasts) and the expression of HPRT (control) in a transplant fragment four weeks later, in Test Example 2. In Fig. 3, the lanes where the preadipocyte field is "-" are experimental results for no preadipocyte addition, and the lanes with "+" are experimental results for preadipocyte addition. In addition, in Fig. 3, lanes displaying "(C)" are the results when Hard Tissue Induction Culture Medium 1 was used, and the lanes not displaying "(C)" are the results when Hard Tissue Induction Culture Medium 2 was used;

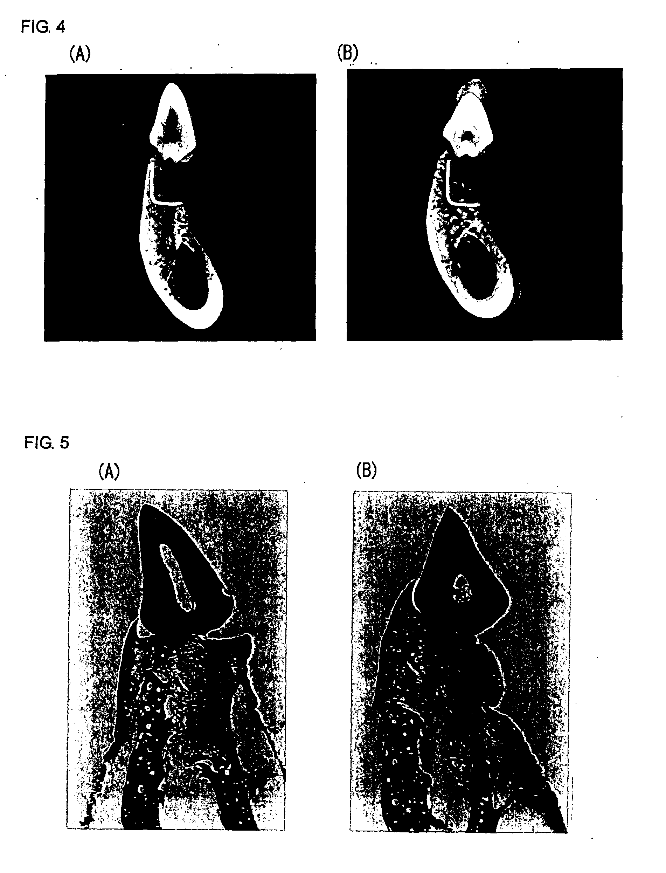

Fig. 4 shows the results of transplantation to the root bifurcation region lesion site of a Beagle dog, which was administered with preadipocyte and fibrin gel, and three-dimensionally analyzing periodontal hard tissue after transplantation, four weeks later, in Test Example 3. The results when preadipocyte and fibrin gel were administered are shown in (A), and the results when fibrin gel alone was administered (control) is shown in (B); and
Fig. 5 shows the results of transplantation to the root bifurcation region lesion site of a Beagle dog, which was administered with preadipocyte and fibrin gel, and observing periodontal hard tissue after transplantation, four weeks later, in Test Example 3. The results when preadipocyte and fibrin gel were administered are shown in (A), and the results when fibrin gel alone was administered (control) is shown in (B).

### BEST MODE FOR CARRYING OUT THE INVENTION

### I. Culture method for causing differentiation into periodontal hard tissue cell

First, the present invention provides a culture method that causes a preadipocyte to differentiate into a periodontal hard tissue cell.

The preadipocyte used in the present invention may be one separated from a mammal, for instance, one separated from a visceral fat tissue or a subcutaneous fat tissue according to a well known method, or may be one that has been induced to differentiate from a stem cell, such as, a mesenchymal stem cell or an ES cell.

The origin of the preadipocyte used in the present invention is also not limited in particular and, for instance, human, mouse, rat, canine, rabbit, feline, bovine, monkey, etc., may be pointed out. Among these, those that are human-derived are preferred in the present invention as clinical application is possible.

In the culture method of the present invention, for the culture medium for culturing the above-mentioned preadipocyte, a culture medium containing at least one member selected from the group consisting of glycerophosphate, and salts thereof (hereinafter, this may be indicated simply with Component (i)), and at least one member selected from the group consisting of ascorbic acid, derivatives thereof and salts thereof (hereinafter, this may be indicated simply with Component (ii)), is used.

Here, as glycerophosphate used as Component (i), it may be either of α-glycerophosphate and β-glycerophosphate, and more preferred is β-glycerophosphate.

In addition, as salts of glycerophosphate used as Component (i), for instance, sodium salt, potassium salt, manganese salt, magnesium salt, iron salt, and the like, may be cited. In the present invention, these salts may be used singly or in combination.

Among Component (i), preferred are β-glycerophosphate and salts thereof, and more preferred is β-glycerophosphate.

As Component (i), the above-mentioned glycerophosphate and salts thereof may be used singly or in combination.

In addition, as derivatives of ascorbic acid used as Component (ii), for instance, ascorbic acid 2-glucoside, ascorbic acid-2 phosphoric acid, ascorbic acid-2 sulfate, ascorbic acid 2,6-dipalmitate, ascorbic acid 6-stearate, ascorbic acid glucosamine, L-dehydroascorbic acid, ascorbic acid 6-palmitate, L-ascorbin tetraisopalmitate and the like, may be cited.

In addition, as salts of ascorbic acid and derivatives thereof used as Component (ii), for instance, sodium salt, potassium salt; calcium salt, magnesium salt and the like, may be cited. In the present invention, these salts may be used singly or combination.

Among Component (ii), preferred is ascorbic acid and salts thereof, and more preferred is ascorbic acid.

As Component (i), although one species of compound may be used singly from among ascorbic acid, derivatives thereof and salts thereof, two or more species of compound may be used in combination from among these.

Although there are no particular limitations as far as the concentrations of the above Components (i) and (ii) in the culture medium used in the present invention, concretely, culture medium containing 0.1 to 100mg/mL of Component (i) and 1 to 1000µg/mL of the Component (ii); preferably a culture medium containing 0.5 to 50mg/mL of Component (i) and 20 to 150µg/mL of Component (ii); more preferably a culture medium containing 0.5 to 10mg/mL of Component (i) and 20 to 80µg/mL of Component (ii) are pointed out. Inclusion at such concentrations of the above-mentioned Components (i) and (ii) in the culture medium allows the induction of differentiation into periodontal hard tissue cell (bone-forming cell, cartilage-forming cell, periodontal ligament-forming cell, and the like) to be carried out all the more efficiently.

In addition, it suffices for the above culture medium that the above-mentioned Components (i) and (ii) have been added in prescribed amounts to a culture medium used for cell culture (suspension culture) in general. For instance, a DMEM culture medium containing 5 to 20 percent in volume of human serum or calf serum albumin to which a prescribed amount of the above Components (i) and (ii) have been added may be cited as a preferred culture medium.

However, it is essential that the above-mentioned culture medium used in the present invention does not contain an adrenal cortical hormone such as dexamethasone in such an effective amount that promotes differentiation of a preadipocyte into a periodontal hard tissue cell. In prior art, in order to cause a stem cell to differentiate into a chondrocyte, a bone-forming cell, a cartilage-forming cell or the like, a culture medium containing an adrenal cortical hormone is used in general. However, in the present invention, differentiation into a periodontal hard tissue cell with respect to a preadipocyte is allowed in a short time and effectively by using a culture medium containing the above-mentioned Components (i) and (ii), and with an amount of adrenal cortical hormone that is less than the effective dose for promoting differentiation of a preadipocyte into a periodontal hard tissue cell, which differs from the observations of prior art techniques. In contrast to this if a prior art culture medium for differentiation promotion containing, together with the above-mentioned Components (i) and (ii), an effective amount of adrenal cortical hormone promoting the differentiation of a preadipocyte into a periodontal hard tissue cell is used, although differentiation of preadipocyte into periodontal hard tissue cell is observed, the rate of differentiation thereof is slow, differentiation into a periodontal hard tissue cell in a short time and effectively is disabled. As the culture media used in the present invention, concretely, a culture medium in which adrenal cortical hormone is less than 10nM, preferably less than 2nM, more preferably less than 1nM and particularly preferably less than 0.5nM, and more preferably, a culture medium substantially not containing an adrenal cortical hormone can be given as examples.

There are no particular limitations regarding the conditions for culturing the preadipocyte, and in general, and it suffices to carry out a culture at 37°C, in the presence of 5% carbon dioxide for 1 to 30 days, and preferably 2 to 14 days. By carrying out a culture in this way differentiation of the above-mentioned preadipocyte into a periodontal hard tissue cell can be induced.

Note that the culture of the preadipocyte may be performed with the preadipocyte in a carrier-attached state in a culture medium, or may be performed with the preadipocyte in a suspended state in a low-adhesion culture dish.

### II. Composition for periodontal hard tissue regeneration

Second, the present invention provides a composition for periodontal hard tissue regeneration containing a scaffold and preadipocytes cultured with a culture medium containing the above-mentioned Components (i) and (ii), but not containing an adrenal cortical hormone in such an amount that promotes differentiation of a preadipocyte into a periodontal hard tissue cell.

The preadipocytes used in the composition for periodontal hard tissue regeneration of the present invention and the culture method thereof are the same as those described in the above article "I. Culture method for causing differentiation into periodontal hard tissue cell".

There is no particular limitation for the scaffold used in the composition for periodontal hard tissue regeneration of the present invention as long as it can be used for the regeneration of a hard tissue and, for instance, materials which are in gel-state or porous and biodegradable or bioresorbable are used. The scaffold may be used singly or in combination.

In particular, in case of using hydroxyapatite and/or fibrin gel as the scaffold, periodontal hard tissue regeneration effect by the preadipocyte is displayed particularly remarkably, thus, hydroxyapatite and/or fibrin gel is a particularly preferred scaffold in the composition for periodontal hard tissue regeneration of the present invention.

As far as the mixing ratio of scaffold contained in the composition for periodontal hard tissue regeneration of the present invention, setting it suitably according to the species or the like of the scaffold is sufficient, and, as one example, proportions of 1 to 4000mg, preferably 5 to 1000mg and preferably 10 to 800mg per 1.0×10⁶ cells of the above-mentioned preadipocytes contained in the composition for periodontal hard tissue regeneration are given as example.

In addition, a pharmaceutically acceptable carrier may be contained in the composition for periodontal hard tissue regeneration of the present invention, in addition to the preadipocyte and scaffold, as necessary. Here, as pharmaceutically acceptable carrier, for instance, physiological saline, buffer solution and the like are given as examples.

In addition, suitable amounts of fibrinogen, thrombin or the like may be added in the composition for periodontal hard tissue regeneration of the present invention, in addition to those described above.

The composition for periodontal hard tissue regeneration of the present invention can be used as a dental therapeutic material, and is useful in particular for the treatment of periodontal diseases accompanied by the destruction of a periodontal hard tissue. Concretely, by administering the composition for periodontal hard tissue regeneration of the present invention to the periodontal-diseased area subjected to the treatment in a suitable amount using a syringe or the like, bone, cartilage and periodontal ligament can be restored comprehensively to regenerate a periodontal hard tissue in its entirety.

In the treatment of periodontal diseases, regarding the dosage of the composition for periodontal hard tissue regeneration of the present invention, setting it suitably according to the extent of the symptoms of the disease, sex and age of the patient, and the like is sufficient and, for instance, it suffices to set the dosage of the composition for periodontal hard tissue regeneration so that the dose of the above-mentioned preadipocyte becomes in the range of 1.0×10⁵ to 1.0×10⁷ cells.

### III. Therapeutic method for periodontal disease

Furthermore, the present invention also provides a "therapeutic method for periodontal disease", which is useful as a regeneration method for periodontal hard tissue. The therapeutic method comprises administering a therapeutically effective amount of scaffold and preadipocytes cultured in a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such an amount that promotes differentiation of a preadipocyte into a periodontal hard tissue cell to a periodontal-diseased area of a patient affected by a periodontal disease.

The preadipocyte used in the composition for periodontal hard tissue regeneration of the present invention and the culture method thereof are the same as those described in the above Article "I. Culture method for causing differentiation into periodontal hard tissue cell". In addition, the scaffolds used in the therapeutic method of the present invention are the same as those described in the above article "II. Composition for periodontal hard tissue regeneration". Furthermore, also regarding the ratio of the scaffold and preadipocytes to be administered, dosage thereof, administration method thereof and target periodontal disease, the contents described in the above article "II. Composition for periodontal hard tissue regeneration" are adopted.

The therapeutic method of the present invention may administer the above-mentioned preadipocytes and the above-mentioned scaffold respectively sequentially or simultaneously to the periodontal-diseased area; however it simpler and preferred to use the composition for periodontal hard tissue regeneration and administer in a periodontal-diseased area.

### EXAMPLES

Hereinafter, the present invention will be described giving examples; however, the present invention is not limited to these examples.

### Test Example 1: Cell differentiation induction

### <Test method>

Preadipocytes collected from a human fat tissue according to well known methods were cultured in a D-MEM culture medium containing 10 mM β-glycerophosphate, 50 µg/ml ascorbic acid and 10 % by volume of FCS (fetal calf serum) (hereinafter abbreviated as "Hard Tissue Induction Culture Medium 1") or a culture medium in which dexamethasone was added to the Hard Tissue Induction Culture Medium 1 so as to obtain 10nM (hereinafter abbreviated as "Hard Tissue Induction Culture Medium 2") at 37°C in the presence of 5% carbon dioxide, at 14 and 21 days of culture, alizarin red staining was carried out on cells in each culture solution according to methods described in the following to evaluate the state of mineralized nodule formation. In addition, at each time point of 0, 7, 14, 21 and 28 days of culture, measurements of DNA amount and alkaline phosphatase (hereinafter abbreviated as ALPase) activity were carried out according to the methods described in the following. Note that in the culture, Hard Tissue Induction Culture Medium 1 and Hard Tissue Induction Culture Medium 2 were replaced every 3 days.

### Alizarin red staining

After removing supernatant of the cell culture, the recovered cells were washed two times with PBS, and then fixed in 100% ethyl alcohol for 10 minutes. With respect to cells fixed in this way, staining in alizarin red S solution for 5 minutes and then washing in distilled water were carried out. The portions stained in alizarin red S were observed with a phase contrast microscope.

### Measurement of ALPase activity

After removing supernatant of the cell culture, the recovered cells were washed two times with PBS, then, sonically disrupted in distilled water with handy sonic, and the liquid fraction was recovered. After sodium paranitrophenol-2-phosphate (pNPP) was added to this liquid fraction so as to obtain 500µM and reacted at 37°C for 30 minutes, the optical density O.D. 415 nm of the same solution was measured to determine the amount of hydrolyzed pNPP. Note that the ALPase activity that hydrolyzes 1 nmol of pNPP in 30 minutes was taken as one unit (U), which was shown as unit values per 1 µg of DNA amount.

### <Test results>

The results of alizarin red staining are shown in Fig. 1 and the ALPase activity measurement results in Fig. 2. From these result, the preadipocytes cultured in Hard Tissue Induction Culture Medium 1 exhibited a high proportion of cells stained with alizarin red even at 14 days of culture, forming mineralized nodules and having differentiated into hard tissue cells (Fig. 1). On the other hand, with the Hard Tissue Induction Culture Medium 2 containing dexamethasone, cells stained with alizarin red were almost not observed at 14 days of culture, revealing that nodule mineralization was not formed (Fig. 1). In addition, with Hard Tissue Induction Culture Medium 1 and Hard Tissue Induction Culture Medium 2, the expression amount of ALPase, which is one indicator for indicating differentiation into a hard tissue cell, demonstrated slightly different behaviors (refer to Fig. 2).

### Test Example 2: in vivo differentiation induction using SCID mouse

Preadipocytes collected from a human fat tissue according to well known methods was cultured using Hard Tissue Induction Culture Medium 1 or Hard Tissue Induction Culture Medium 2 at 37°C in the presence of 5% carbon dioxide and the cells were recovered at 7 days of culture. After 1.5x10⁶ cells of the recovered cells and 40 mg of hydroxyapatite or β-TCP were mixed and cultured at 37°C for 90 minutes, 15 µl each of fibrinogen and thrombin were added and transplanted under the dorsal skin of a six week-old SCID mouse. The transplant was taken out at four weeks after transplantation, and the amount of human osteocalcin mRNA expressed in the transplant was measured by the RT-PCR method. Note that as a control, measurements were carried out similarly with respect to the mRNA expression amount for Hypoxanthine phosphoribosyl transferase (HPRT).

The results are shown in Fig. 3. As is apparent from Fig. 3, when preadipocytes cultured using Hard Tissue Induction Culture Medium 1 were transplanted using hydroxyapatite as a scaffold, a remarkable human osteocalcin (differentiation marker for osteoblasts) expression was observed. From this result, it has become apparent that the regeneration of periodontal hard tissue by preadipocytes cultured using Hard Tissue Induction Culture Medium 1 may be carried out all the more effectively by using preadipocytes and hydroxyapatite in combination.

### Test Example 3: Transplantation of preadipocytes to a Beagle dog periodontal disease model and analysis thereof

A mandibular artificial lesion of the secondary root bifurcation region of a two year-old Beagle dog was created, and simultaneously, dorsal mesogastrium was collected from the abdomen. Preadipocytes were isolated from the collected dorsal mesogastrium and conserved. The isolated and conserved preadipocytes were cultured in Hard Tissue Induction Culture Medium 1 at 37°C in the presence of 5% carbon dioxide, and the cells were recovered at 7 days of culture. Mixed were 1.5×10⁵ cells of the recovered cells and 100µl of fibrin gel, and this was transplanted on the experimental side of the root bifurcation region lesion of the above-mentioned Beagle dog at for weeks after the operation. In addition, fibrin gel alone was packed on the control side of the root bifurcation region lesion. The mandible was collected four weeks after the transplantation, and the periodontal hard tissue was photographed by microCT to evaluate the extent of regeneration thereof.

The results are shown in Figs. 4 and 5. In Fig. 4, the results when preadipocyte and fibrin gel were administered are shown in (A), and the results when fibrin gel alone was administered (control) is shown in (B). In addition, in Fig. 5, the results when preadipocytes and fibrin gel were administered are shown in (A), and the results when fibrin gel alone was administered (control) is shown in (B). From these results, it was revealed that, along with bone regeneration, regeneration of cartilage and periodontal ligament was also observed in the root bifurcation region lesion site where preadipocytes and fibrin gel were administered, and that the root bifurcation region lesion site was regenerated as a periodontal hard tissue. On the other hand, regeneration of periodontal hard tissue was not observed when simply fibrin gel was administered.

## Claims

1. A culture method for causing a preadipocyte to differentiate into a periodontal hard tissue cell, wherein a preadipocyte is cultured with a culture medium containing (i) at least one mamber selected from the group consisting of glycerophosphate and salts thereof, and (ii) at least one member selected from the group consisting of ascorbic acid, derivatives thereof and salts thereof, but not containing an adrenal cortical hormone in such an amount that promotes differentiation of a preadipocyte into a periodontal hard tissue cell.

2. The method according to Claim 1, wherein the preadipocyte is human-derived.

3. The method according to Claim 1, wherein Component (i) is β glycerophosphate and Component (ii) is ascorbic acid.

4. The method according to Claim 1, wherein the culture medium contains 0.1 to 100 mg/mL of Component (i) and 1 to 1000 µg/mL of Component (ii).

5. The method according to Claim 1, wherein the culture medium contains the adrenal cortical hormone in an amount of less than 10 nM.

6. A composition for periodontal hard tissue regeneration, containing a scaffold, and preadipocytes cultured with a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such an amount that promotes differentiation of a preadipocyte into a periodontal hard tissue cell.

7. The composition for periodontal hard tissue regeneration according to Claim 6, wherein the scaffold is hydroxyapatite and/or fibrin gel.

8. The composition for periodontal hard tissue regeneration according to Claim 6, wherein the preadipocyte is human-derived.

9. The composition for periodontal hard tissue regeneration according to Claim 6, containing 1 to 4000mg of the scahold per 1.0×10⁶ cells of the preadipocytes.

10. A therapeutic method for periodontal disease, comprising administering a therapeutically effective amount of scaffold and preadipocytes cultured with a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such an amount that promotes differentiation of a preadipocyte into a periodontal hard tissue cell to a periodontal-diseased area of a patient affected by a periodontal disease.

11. The therapeutic method for periodontal disease according to Claim 10, wherein a therapeutically effective amount of the composition for periodontal hard tissue regeneration of Claim 6 is administered to a periodontal-diseased area of a patient affected by a periodontal disease.

12. Use of a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such an amount that promotes differentiation of a preadipocyte into a periodontal hard tissue cell, for causing a preadipocyte to differentiate into a periodontal hard tissue cell.

13. Use of a scaffold and preadipocyte cultured with a culture medium containing (i) at least one member selected from the group consisting of glycerophosphate and salts thereof, and (ii) at least one member selected from the group consisting of ascorbic acid, derivatives thereof and salts thereof, but not containing an adrenal cortical hormone in such an amount that promotes differentiation of a preadipocyte into a periodontal hard tissue cell, for preparing a composition for periodontal hard tissue regeneration.
